# EUROPÄISCHE PATENTSCHRIFT

(11) **EP 1 366 774 B1**
(45) Veröffentlichungstag und Bekanntmachung des Hinweises auf die Patenterteilung: **04.07.2007**
(21) Anmeldenummer: 03009377.7
(22) Anmeldetag: 25.04.2003
(51) Int. Cl.: A61L 24/02, A61L 24/04, A61K 49/04

(54) **Knochenzementmischung und Röntgenkontrastmittel**
Bone cement and X-ray contrast agent
Ciment osseux et agent de contraste radiographique

(30) Priorität: 29.05.2002 DE 10224346
(43) Veröffentlichungstag der Anmeldung: 03.12.2003
(60) Teilanmeldung: 07000986.5
(73) Patentinhaber: Heraeus Kulzer GmbH, 63450 Hanau (DE)
(72) Erfinder: Kühn, Klaus-Dieter, Dr., 35041 Marburg (DE)
(74) Vertreter: Kühn, Hans-Christian

(56) Entgegenhaltungen:
- EP-A- 0 041 614
- WO-A-00/57932
- MISRA D N: "Adsorption of zirconyl salts and their acids on hydroxyapatite: use of the salts as coupling agents to dental polymer composites." JOURNAL OF DENTAL RESEARCH. UNITED STATES DEC 1985, Bd. 64, Nr. 12, Dezember 1985 (1985-12), Seiten 1405-1408, XP009013841 ISSN: 0022-0345
- KIM H Y ET AL: "Improvement of fatigue properties of poly(methyl methacrylate) bone cement by means of plasma surface treatment of fillers" J BIOMED MATER RES;JOURNAL OF BIOMEDICAL MATERIALS RESEARCH 1999 JOHN WILEY & SONS INC, NEW YORK, NY, USA, Bd. 48, Nr. 2, 1999, Seiten 135-142, XP002248232
- SKRTIC DRAGO ET AL: "Physicochemical evaluation of bioactive polymeric composites based on hybrid amorphous calcium phosphates." JOURNAL OF BIOMEDICAL MATERIALS RESEARCH, Bd. 53, Nr. 4, 2000, Seiten 381-391, XP002248233 ISSN: 0021-9304
- KJELLSON F ET AL: "Tensile properties of a bone cement containing non-ionic contrast media" JOURNAL OF MATERIALS SCIENCE: MATERIALS IN MEDICINE 2001 NETHERLANDS, Bd. 12, Nr. 10-12, 2001, Seiten 889-894, XP009013837 ISSN: 0957-4530

## Beschreibung

Die Erfindung betrifft die Verwendung einer Polymerkomponente und einer Monomerkomponente sowie die Verwendung eines Röntgenkontrastmittels.

Unter Knochenzementmischung im Sinne der Erfindung ist ein Zweikomponentensystem zu verstehen, bei dem eine Komponente als pulverförmige Polymerkomponente und eine zweite Komponente als flüssige Monomerkomponente vorliegen. Beide Komponenten werden in der Regel erst unmittelbar vor der Verwendung miteinander gemischt.

Knochenzemente, insbesondere solche, die zur Verankerung künstlicher Gelenke verwendet werden, enthalten Röntgenkontrastmittel zur klinischen Verlaufskontrolle. Diese Röntgenkontrastmittel erlauben dem Chirurgen eine sichere Überprüfung des Implantats und eine frühzeitige Diagnose möglicher Komplikationen, wie z.B. Lockerungen. Knochenzemente sind chemische Kunststoffe auf Acrylatbasis (beispielsweise Palacos TM R der Heraeus Kulzer GmbH & Co. KG). Sie werden als Zweikomponenten-Systeme angeboten mit einer pulverförmigen Polymermischung und einem flüssigen Monomer. Als Kontrastmittel werden dabei der Polymermischung Zirkondioxid oder Bariumsulfat zugesetzt. Diese Röntgenkontrastmittel werden allerdings nicht in die Polymerkette eingebunden und gelten deshalb als mögliche Ursache für Mikrorisse im Zementmantel. Zirkondioxid kann an Grenzflächen unter Umständen abrasiv wirken.

EP 41 614 beschreibt beschichtetes BaSO4-Pulver für Zahnfüllmassen, das durch Dispergieren und Zugabe von Polymerlösung hergestellt wird. EP 89782 beschreibt beschichtetes Bariumsulfat, das durch Mischen von PMMA Teilchen und BaSO4 Partikeln mit HEMA und anschliessendes Erhitzen hergestellt wird. JP 06024927 betrifft Polymer-Füller Composites, die durch Polymerisieren von Säuremonomer und weiterem ungesättigten Monomer in Gegenwart von Röntgenkontrastpulver hergestellt werden.

J. Dent. Res. 64(12), 1405-8 (1985) beschreibt die Adsorption von Zirconylmethacrylat auf synthetisch hergestelltem Hydroxylapatit. Ziel dabei war es, diese Verbindung mit anorganischem Anteil und Doppelbindungen als Haftvermittler zu nutzen. Damit kann eine festere Verbindung der (natürlichen) Apatitoberfläche mit den Methacrylaten von Dentalmaterialien erreicht werden.

In J. Biomed. Mat. Res. 48 (2), 135-42 (1999) geht es um Plasmaoberflächenbehandlung des Füllstoffs Zr02 in Knochenzement. Gemäß diesem Dokument wird dadurch das Polymerisationsverhalten des MMA verändert. Es wird eine chemische Bindung zwischen der Füllstoffoberfläche und der PMMA Matrix vermutet, aber nicht belegt, und es handelt sich nicht um Zr enthaltende Methacrylate.

J. Biomed. Mat. Res. 2001 (889-94) D5 beschreibt Composite, gebildet aus diversen Monomeren und Zirkonylmethacrylat (Tabelle III). Dort wird ein bioaktiver Polymer-Komposit als Zahnfüllmaterial beschrieben, der amorphes Calciumphosphat als Füllmaterial enthält. Zur Anbindung der Polymere (Resins) an das amorphe Calciumphosphat wurde bei der Synthese des amorphen Calciumphosphates Tetraethoxysilan und Zirkonylchlorid zugesetzt. Dadurch wurde letztendlich eine Hydrophobierung des amorphen Calciumphosphates erreicht. Hinweise auf das Einpolymerisieren von Zirkonylmethacrylat, um Polymere mit röntgenopaken Eigenschaften zu erhalten, finden sich nicht.

Der Erfindung liegt die Aufgabe zugrunde, ein verbessertes Röntgenkontrastmittel und einen diesen enthaltenden Knochenzement zur Verfügung zu stellen.

Erfindungsgemäss wird die Aufgabe durch die Merkmale des unabhängigen Anspruchs gelöst.

Das Röntgenkontrastmittel ist ausgebildet als Polymer oder Copolymer mit daran chemisch gebundenem Zirkonyl-di-methacrylat und/oder andere chemisch gebündenes Zirkonium und/oder andere chemisch gebündene röntgenopake Elemente enthaltenden Methacrylate.

Derartige Röntgenkontrastmittel enthaltende Knochenzementmischungen, die eine Polymerkomponente und eine Monomerkomponente aufweisen, wobei die Polymerkomponente Polymere und/oder Copolymere aufweist, werden erfindungsgemäss dadurch hergestellt, dass während der Herstellung der Polymere und/oder Copolymere ein chemisch gebündene röntgenopake Elemente enthaltendes Polymer und/oder Monomer zugesetzt werden. Wenn das Röntgenkontrastmittel durch Einpolymerisieren von Zirkonyl-di-methacrylat und/oder andere Zirkonium und/oder andere chemisch gebündene röntgenopake Elemente enthaltende Methacrylate in ein Polymer oder Copolymer hergestellt wird, dann weisen derart hergestellte Röntgenkontrastmittel bzw. Knochenzementmischungen keine mineralischen Bestandteile auf, die oben genannten Nachteile hervorrufen, da das resultierende erfindungsgemässe Röntgenkontrastmittel als Polymer oder Copolymer ausgebildet ist.

Es hat sich als zweckmässig erwiesen, dass das Röntgenkontrastmittel chemisch gebündenes Zirkonium und/oder Barium und/oder andere chemisch gebündene röntgenopake Elemente, insbesondere Zirkonyl-di-methacrylat und/oder andere Zirkomum und/oder andere röntgenopake Elemente enthaltende Methacrylate enthält.

Insbesondere wird Zirkonyl-di-methacrylat in das Polymer oder Copolymer einpolymerisiert und der Polymerkomponente zugefügt. Barium und/oder Zirkonium und/oder andere chemisch gebündene röntgenopake Elemente enthaltende Polymere oder Copolymere können gemäss der Erfindung als Röntgenkontrastmittel in Knochenzementmischungen verwendet werden.

Nachfolgend werden Ausführungsbeispiele der Erfindung beschrieben.

Als Monomerkomponente wird eine für Knochenzemente übliche Monomerkomponente verwendet. Die Polymerkomponente wird hergestellt aus einem Copolymer mit 20% Zirkonyl-di-methacrylat und einem Initiator, der im Anteil von etwa 1% an der Polymerkomponente vorliegt.

In einem zweiten Beispiel wird die Polymerkomponente gebildet aus einem Copolymer mit 40% Zirkonyl-di-methacrylat, einem Polymer aus Polymethylmethacrylat oder Copolymeren und einem Initiator, wobei Zirkonyl-di-methacrylat enthaltendes Copolymer im Anteil von 50%, das Polymer im Anteil von 49% und der Initiator im Anteil von 1% an der Gesamtkomponente vorliegt.

Ein drittes Beispiel weist die gleichen Bestandteile wie das zweite Beispiel auf, wobei das Copolymer einen 80%igen Zirkonyl-di-methacrylat-Anteil aufweist und in der Gesamtmischung der Polymerkomponente mit 25% vorliegt, das Polymer liegt mit 74% vor und der Initiator mit 1 %.

Die Opazität des Materials lässt sich durch den Gehalt von Zirkonium (bzw. Barium) einstellen.

Durch das Hinzufügen von nicht röntgenopakem (Co)polymer lässt sich die Röntgenopazität der Knochenzementmischung so einstellen, dass die Röntgenopazität einer Menge von 5-45 %, vorzugsweise 8-16 %, Röntgenkontrastmittel in der Polymerkomponente des Knochenzements entspricht.

## Patentansprüche

1. Verwendung eines Polymer oder Copolymer mit daran chemisch gebundene Zirkonyl-di-methacrylat und/oder anderen, einpolymerisierten, chemisch gebundenes, röntgenopake Elemente enthaltenden Meth-acrylaten und/oder anderen, einpolymerisierten, chemisch gebundenes Zirkonium enthaltenden Meth-acrylaten als Röntgenkontrastmittel.

2. Verwendung nach Anspruch 1 in Knochenzementmischungen.

## Claims

1. Use of a polymer or copolymer with zirconyl dimethacrylate chemically bonded thereto and/or other methacrylates incorporated in the form of polymerized units and containing chemically bonded X-ray-opaque elements and/or other methacrylates incorporated in the form of polymerized units and containing chemically bonded zirconium as an X-ray contrast agent.

2. Use according to Claim 1 in bone cement mixtures.

## Revendications

1. Utilisation d'un polymère ou copolymère avec un diméthacrylate de zirconyle lié chimiquement, et/ou d'autres méthacrylates contenant des éléments opaques aux rayons X liés chimiquement polymérisés et/ou d'autres méthacrylates contenant du zirconium liés chimiquement polymérisés comme agent de contraste pour rayons X.

2. Utilisation selon la revendication 1 dans des mélanges pour ciment osseux.
